# EUROPEAN PATENT SPECIFICATION

(11) **EP 2 293 828 B1**
(45) Date of publication and mention of the grant of the patent: **13.05.2015**
(21) Application number: 09759368.5
(22) Date of filing: 03.06.2009
(51) Int. Cl.: A61M 1/02

(54) **BLOOD INFUSION MANAGEMENT SYSTEM AND METHOD**
SYSTEM UND VERFAHREN ZUR VERWALTUNG VON BLUTINFUSIONEN
SYSTÈME ET PROCÉDÉ DE GESTION DE PERFUSION DE SANG

(30) Priority: 04.06.2008 US 133346
(43) Date of publication of application: 16.03.2011
(73) Proprietor: CareFusion 303, Inc., San Diego, CA 92130 (US)
(72) Inventor: SMITH, Nancy, Lorton VA 22079 (US); SINHA, Manish, Chandigarh 160022 (IN)
(74) Representative: Richards, John
(86) International application number: PCT/US2009/046170
(87) International publication number: WO 2009/149209

(56) References cited:
- WO-A-2006/059156
- WO-A-2007/014147
- WO-A-2008/008281

## Description

### FIELD

The present disclosure generally relates to blood infusion management and, in particular, relates to blood infusion management systems and methods including rapid blood infusion.

### BACKGROUND

Blood and blood product transfusion errors are among the most serious types of medical errors, and present serious risks of harm including death. Approximately 60-70% of all blood products in an acute care setting are transfused in emergent environments, such as operating suites, emergency departments, and critical care areas. Nurses in these areas are sometimes asked to sign blood slips without having had the chance to verify the patient's identity, without checking pertinent blood bank information, or even without the chance to check the patient's name bracelet.

Root causes for blood transfusion errors, as reported by the Joint Commission on the Accreditation of Healthcare Organizations, include: incomplete patient verification, the storage of blood for multiple surgical patients in the same refrigeration unit, incomplete communication, and errors related to patient, specimen, and blood label identification.

WO/2006/059156 discloses a system for matching blood information with transfusion information prior to a blood transfusion. The system includes a portable hand-held device including code recognition means, printing means, and programmed computer means for comparing unique codes contained within first and second identifier means.

WO/2008/008281 discloses methods and systems for evaluating a fluid transfer event between a parenteral fluid delivery and a patient. The evaluation of the fluid transfer event can be prospective, real-time and/or historic, as desired, and take a variety of different formats.

### SUMMARY

According to certain exemplary embodiments of the inventions disclosed herein, a management system, as claimed, for blood infusion during emergent situations is provided. A scanner provides scan data from individual blood units and a patient identifier particular to an individual patient to a processor. A display, such as an LCD screen or a computer monitor, visually displays the status of individual blood unit(s) within a blood infusion protocol. The processor, which may be a medical-grade computer, runs the blood infusion protocol, provides data to the display for visual display of the blood infusion protocol (including the status of at least one individual blood unit), receives and processes the scan data from the scanner, and aborts the blood infusion protocol if the processed scan data indicates that any of the individual blood units do not match the patient identifier. The blood infusion protocol requires at least two verification scans of an individual blood unit where the individual blood unit must match the patient identifier to acquire a transfusing status on the display for an individual blood unit.

According to certain exemplary embodiments, a method, as claimed, for authorizing blood infusion is provided, and includes deriving scan data from individual blood units and a patient identifier particular to an individual patient. The blood infusion protocol requires at least two verification scans of an individual blood unit where the individual blood unit must match the patient identifier to acquire a transfusing status on the display for the individual blood unit.

According to certain exemplary embodiments, a computer-readable medium, as claimed, such as a CD-ROM or a computer disk, is encoded with computer-executable instructions for causing a processor to execute instructions for a blood infusion protocol by performing the steps of deriving scan data from individual blood units and a patient identifier particular to an individual patient, visually displaying on a display at least one individual blood unit status within a blood infusion protocol, running the blood infusion protocol, providing data to the display for visual display of the blood infusion protocol, including the at least one individual blood unit status, receiving and processing the scan data from the scanner, and aborting the blood infusion protocol if the processed scan data indicates that any of the individual blood units do not match the patient identifier. The blood infusion protocol requires at least two verification scans of an individual blood unit, where the individual blood unit must match the patient identifier to acquire a transfusing status on the display for the individual blood unit.

According to certain exemplary embodiments, various means or steps plus functions are provided for carrying out the above-described systems, methods, and sets of computer-executable instructions, as described herein.

In the following description, reference is made to the accompanying attachment that forms a part thereof, and in which are shown by way of illustration specific embodiments in which the inventions may be practiced. It is to be understood that other embodiments may be utilized and changes may be made without departing from the scope of the present inventions. Both the foregoing general description and the following detailed description are exemplary and explanatory, and are intended to provide further explanation of the discussed embodiments as claimed.

### BRIEF DESCRIPTION OF THE DRAWINGS

The accompanying drawings, which are included to provide further understanding and are incorporated in and constitute a part of this specification, illustrate disclosed embodiments and together with the description serve to explain the principles of the disclosed embodiments. In the drawings:
Figure 1 is a block diagram of an exemplary embodiment including a blood infusion management system, and/or means for same;
Figure 2 is a block diagram of an exemplary embodiment including instruction steps that are capable of being executed by a computer, and/or means for same;
Figure 3 is a flow diagram of an exemplary embodiment illustrating a process or protocol for blood infusion management in certain care environments, such as an operating room;
Figure 4 is a flow diagram of an exemplary embodiment illustrating a process or protocol for blood infusion management in certain care environments, such as an emergent location;
Figure 5 is a flow diagram of aspects of exemplary embodiments illustrating a documentation process that may be provided to medical professional users at the end of the blood transfusion processes described herein, for example, for those processes described in relation to Figures 3 and 4;
Figures 6 - 13 are screenshots of configuration screens for an exemplary embodiment;
Figures 14 and 15 are screenshots of rapid infusion order screens for an exemplary embodiment;
Figures 16 - 20 are screenshots of a rapid infusion process screen for an exemplary embodiment;
Figure 21 is a screenshot of a rapid infusion blood product selection screen for an exemplary embodiment;
Figure 22 is a screenshot of a rapid infusion documentation screen for an exemplary embodiment;
Figure 23 is a screenshot of a rapid infusion witness documentation screen for an exemplary embodiment;
Figure 24 is a screenshot of a rapid infusion volume documentation screen for an exemplary embodiment; and
Figures 25 - 41 are screenshots of a standard infusion documentation screen for a prior art blood infusion protocol.

### DETAILED DESCRIPTION

The following description of illustrative non-limiting embodiments of the invention discloses specific configurations and components. However, the embodiments are merely examples of the present invention, and thus, the specific features described below are merely used to describe such embodiments to provide an overall understanding of the present invention. One skilled in the art readily recognizes that the present invention is not limited to the specific embodiments described below. Furthermore, certain descriptions of various configurations and components of the present invention that are known to one skilled in the art are omitted for the sake of clarity and brevity. Further, while the term "embodiment" may be used to described certain aspects of the invention, the term "embodiment" should not be construed to mean that those aspects discussed apply merely to that embodiment, but that all aspects or some aspects of the disclosed invention may apply to all embodiments, or some embodiments.

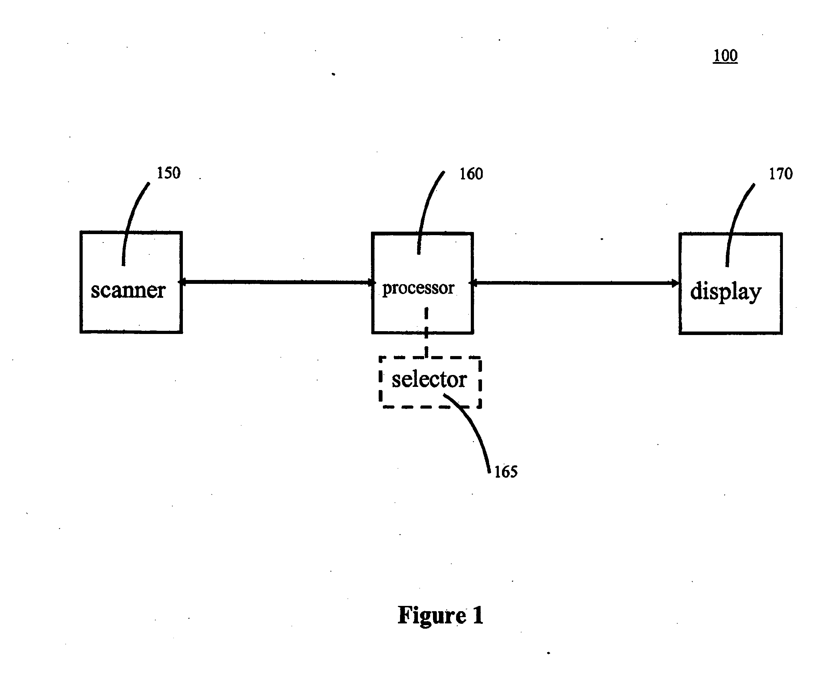

Figure 1 is a block diagram of an exemplary embodiment including a blood infusion management system 100, and/or means for same. The figure includes a scanner unit (or means for scanning) 150. The scanner is in communicative contact with processor unit (or means for processing)160. The processor is in communicative contact with a display unit (or means for displaying) 170. Communications between the scanner 150, the display 170, and the processor 160 may be one-way or bi-directional. In certain exemplary embodiments, the scanner 150, the processor 160, and the display 170 are separate modules. For instance, the scanner 150 may be a hand-held scanner or a finger-held scanning wand, the processor 160 may be a medical-grade computer including a processor, volatile and non-volatile memory, and a video-output card, and the display 170 may be an flat panel display (such as an liquid crystal display (LCD) or plasma display), or a television (TV) screen, or a cathode ray tube (CRT). These are all examples only, as other types of scanners, processors, and displays may be employed.

In certain exemplary embodiments, the scanner 150, the processor 160, and the display 170 may comprise three or fewer modules, for instance, the scanner 150 may be coupled in a hand-held Personal Digital Assistant (PDA) or like device that includes an LCD screen that functions as display 170, and that also includes a processor 160. In certain exemplary embodiments, the scanner 150 and the display 170 may be coupled as a single module that is in communicative contact with the processor 160, for example where the processor is a laptop computer coupled with a medical-grade stainless steel cart for portability. Display 170 may include multiple means for display, such as both of a laptop computer screen and a PDA screen for simultaneous display.

Processor 160 is configured to at least receive data from scanner 150, but may also be configured to transmit data to same. Processor 160 is configured to at least transmit data to display 170, but may also be configured to receive data from same. Communication between the processor 160 and the scanner 150 and the display 170 may be continuous, or the processor 160 may communicate data as necessary. Communication between the processor 160 and the display 170 and scanner 150 may be achieved via a communication layer that enables data transmissions. Example communications include serial communication interfaces such as RS-232, Ethernet, Universal Serial Bus (USB), and wireless interfaces such as radio frequency (RF), infrared, Bluetooth^{®}, and IEEE 802.11x.

Any of scanner 150, processor 160, and display 170 may comprise onboard memory. Memory can be either non-volatile storage (e.g., read-only memory, flash memory, magnetic media, etc.), volatile storage (e.g., random-access memory), or both.

In certain exemplary embodiments, the scanner 150 is utilized to scan a patient's identification bracelet and at least one blood product unit particular to the patient. The scanner 150 may be one of a laser scan (useful for reading bar codes) and a radio frequency scan (useful for reading radio frequency tags), or other suitable type of reader. The scanner 150 derives scan data from the patient identification bracelet and the at least one blood product unit particular to the patient. The scan data is then communicated to the processor 150.

Processor 160 may then store the scan data in memory for later processing, but typically will process the scan data in real time, or substantially in real time, in a blood infusion protocol run by processor unit 160. In certain exemplary embodiments, multiple blood infusion protocols are provided for selection by processing unit 160. For example, the processor unit 160 may be provided with a selector unit 165. The selector unit 165 may comprise a software configuration screen that includes selection between service level settings. The service level settings may be defaulted to a particular protocol and/or may be configured to provide user-selection between multiple blood infusion protocols.

For example, when the blood infusion management system 100 is first introduced to a particular setting, a configuration screen is utilized to determine what types of blood infusion protocols will be allowed based on the particular setting. For instance, whether the setting is anticipated to have a heightened sense of urgency for patients, such as an emergency room, or operating room, or whether the setting is anticipated to have less than a heightened sense of urgency, and user status. For example, a nurse-in-charge may be provided with the ability to choose between protocols based on the type of infusion needed by the medical professionals at that particular time. Certain exemplary embodiments are adaptive to the constantly changing environments faced by the medical community. That is, through use of a service level setting and a user-selection, a level of blood infusion service is adaptively managed based on the needs of the moment. For example, a nurse-in-charge may select a protocol for one of an emergent location and an operating room location, where the service level has been pre-configured to adaptively provide the selection between the two protocols and the final determination is made by a qualified user.

Examples of multiple blood infusion protocols include rapid infusion protocols and standard infusion protocols. Rapid infusion protocols comprise sets of instructions and approvals or denials of blood infusion during crisis situations, such as in emergent environments and operating room environments, or generally those environments requiring a heightened sense of urgency, and may allow for infusion documentation. For example, infusion documentation may include an infusion checklist, patient vital signs, witness verification, and volume levels, after the process of infusion. The rapid infusion protocol may be displayed on the display 170 as having a red border for all screens to provide an instant visual affirmation to a user that the rapid infusion protocol is in effect.

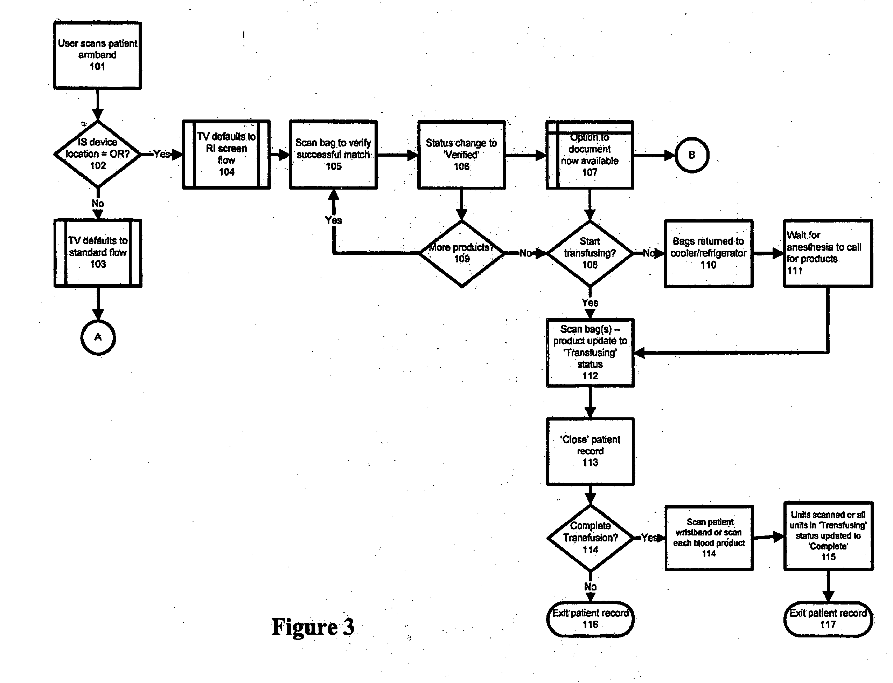

A general overview of an exemplary process will now be described, followed by a more detailed description with respect to Figure 3. Standard infusion protocols comprise sets of instructions and approvals or denials of blood infusion during non-emergency situations, and require infusion documentation (for example, infusion checklist, patient vital signs, witness verification, and volume) during the process of infusion. The standard infusion protocol may be displayed on the display 170 as having a border on all screens that is not red to provide instant visual affirmation to a user that the standard infusion protocol is in effect.

Processor unit 160 runs the selected blood infusion protocol and provides the display with visual display information including the status of individual blood units. Near the beginning of the protocol, the processor unit 160 evaluates whether the scan data consists of a match between a patient identifier (such as a barcode on a patient bracelet) and each individual blood unit that is intended to be used for the patient for the instant infusion. That is, each individual blood unit that is intended for the patient, and the patient identifier, are scanned by scanner 150 to derive initial verification scan data.

The initial verification scan data is communicated to the processor unit 160, and the processor unit 160 evaluates whether the patient identifier and the blood units are a match. For instance, evaluation is made as to blood type between the patient and the blood units, and if both need to be - and actually are - 'A positive,' and if the blood product is the correct type for the intended procedure (for instance, if the blood units are blood platelets or red blood cells, for example), then the processor unit 160 sends a signal to the display 170 that changes the status of the blood units in question within the displayed blood infusion protocol. For instance, the fill (or background) color of the screenshot representing the individual blood unit may change to red and 'VERIFIED' may be displayed within the screenshot for the individual blood unit on the display 170.

Initial verification scans are provided for each individual blood unit, and each musf match the initial scan of the patient identifier to achieve a 'VERIFIED' status on the display 170. Once all blood units have had an initial verification scan, and if all of the blood units match, then the blood units may be provided to a blood unit storage unit (not shown) for expected use. If needed immediately, or once retrieved from the blood storage unit for use, a second verification scan of each individual blood unit is required prior to infusion of the blood product, where each individual blood unit must once again match the initial scan of the patient identifier.

Notably, it is only after the second verification scan with the individual blood unit matching the patient identifier and blood unit type, that the processor unit 160 provides the display 170 with a 'TRANSFUSING' status in the screenshot for the individual blood unit in question. Therefore, each individual blood unit has had both a matching first verification scan and a matching second verification scan, and it is only after both of these two verification scans that individual blood product units may be infused to a patient.

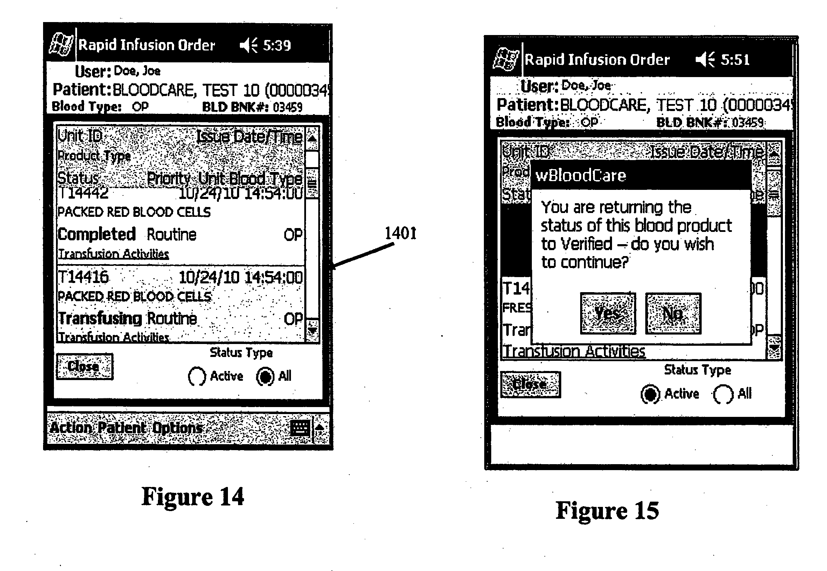

In certain exemplary embodiments, whenever any of the two verification scans do not match the patient identifier, the processor unit 160 aborts the blood infusion protocol. In certain exemplary embodiments, at any point where any of the two verification scans do not match the patient identifier, the display is provided with a protocol abortion as to the individual blood unit that does not match. In certain exemplary embodiments, at any point where an individual blood unit does not match the patient identifier, the blood infusion protocol is allowed to continue with compliant, matching individual blood units, but the non-matching blood unit status is reverted to non-verified and the screenshot for the non-matching individual blood unit loses its red fill, or red background, and the user is provided with the option of returning to the point of having to perform two verification scans in order to reach a 'TRANSFUSING' status for that individual blood unit. An example of a blood unit with a display status of 'VERIFIED' and with a red background is shown in Figure 15. The blood product unit displayed with the red background in Figure 15 has had one verification scan where the blood product unit matched the patient identifier. If the noted blood product unit failed to match the patient identifier for the required second verification scan, that blood product unit would loose its red background and attendant 'VERIFIED' status.

In many instances, if an individual blood unit fails to match a patient identifier after a second verification scan, that individual blood unit will be segregated until the emergency situation has been handled, and then a determination will be made as to why the blood unit failed to match the second verification scan. Such an instance could represent a failure to properly segregate blood units for separate patients, or another type of human error.

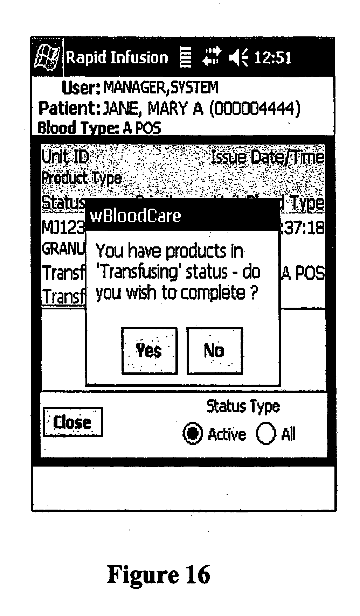

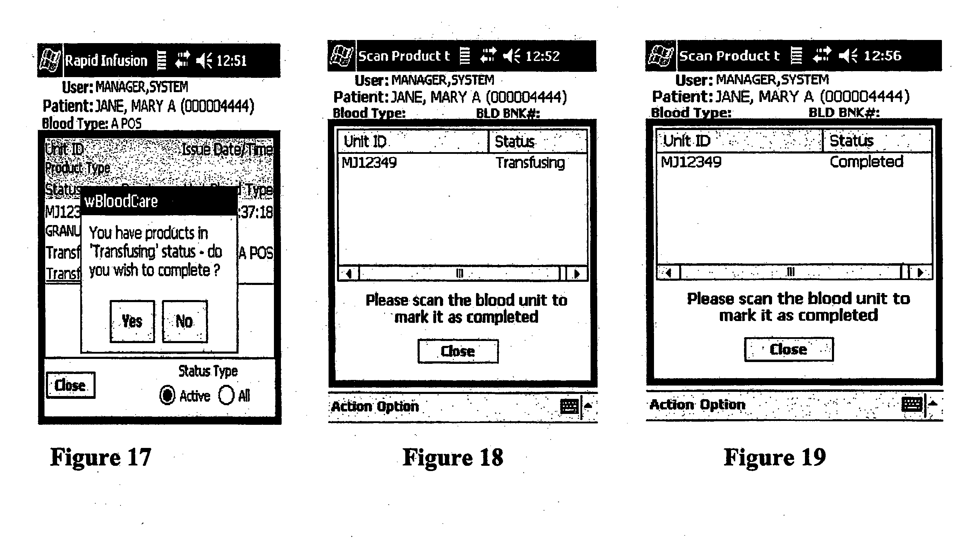

Once the medical professional has determined that the patient no longer needs continued blood infusion, a second scan of the patient's identifier may be made to bring all previously 'TRANSFUSING' individual blood units up-to-date with a 'COMPLETE' status as shown by a change in the screenshot on the display 170 for the blood units in question. This is shown, for example, by Figure 16, where the screenshot notifies the user that there are still blood product units in a 'TRANSFUSING' status, and inquires if the user wishes to proceed. Alternatively, the medical professional may scan each individual blood unit a third time to individually bring each individual blood unit up-to-date with a 'COMPLETE' status as shown by a change in the screenshot on the display 170 for the blood unit in question. For instance, the screen shot will change from the 'TRANSFUSING' status shown in Figure 14 for blood product unit T14416 to a 'COMPLETE,' or 'COMPLETED' status, for example, as shown by blood product unit T14442 (also shown in Figure 14). Additional figures showing screenshots for scanning of individual blood product units to achieve a 'COMPLETE' status is shown in Figures 18 and 19.

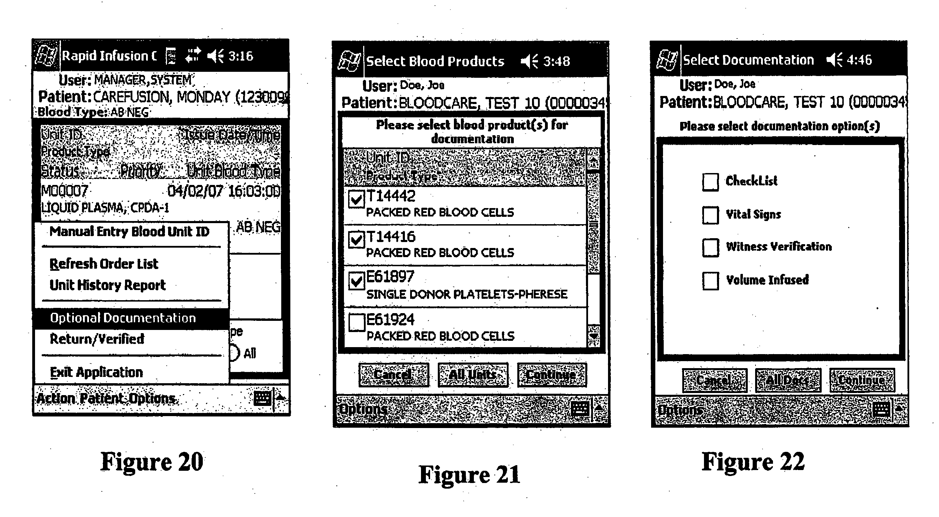

At the point where all individual blood units have attained a 'COMPLETE' status, the blood infusion protocol is configured to provide user access to infusion documentation, for instance as shown in Figures 20 - 24. The documentation may include patient vital signs, witness verification, blood unit volume, and/or a post-procedure blood infusion checklist. In certain exemplary embodiments, the processor unit 160 may be configured to require that the blood infusion documentation must be completed within a certain time frame after an individual blood unit achieves a 'COMPLETE' status, for instance, within 4 hours of the 'COMPLETE' status, or within 12 or 24 hours of the 'COMPLETE' status.

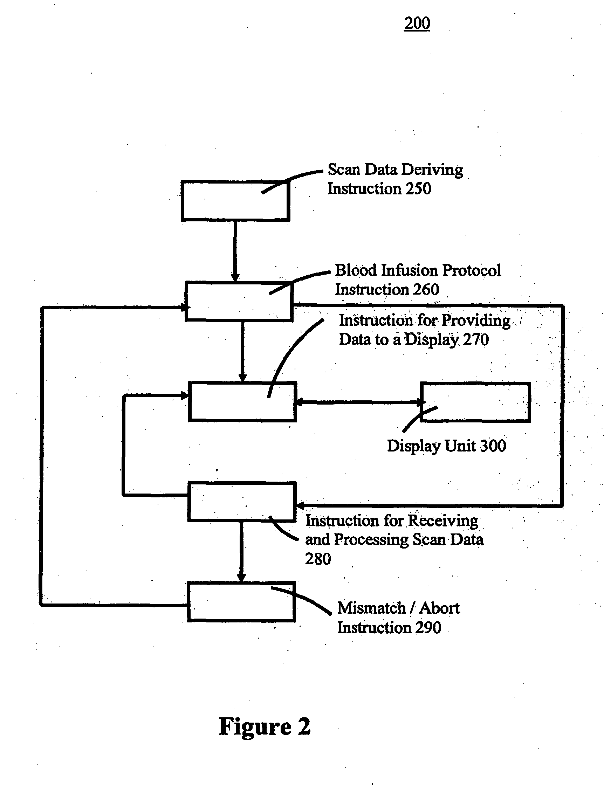

Figure 2 is a block diagram of an exemplary embodiment including instruction steps/means 200 that are capable of being executed by a computer, and/or means for same. The figure includes a scan data deriving instruction 250, a blood infusion protocol instruction 260, an instruction for providing data to a display 270, an instruction for receiving and processing scan data 280, a mismatch/abort instruction 290, and a display unit 300.

The instruction steps/means 200 may be stored in a computer-readable format, such as in digital computer code, and may be stored on a computer-readable product such as a CD-ROM, a computer hard disk, a computer floppy disk, a zip file, or in other forms of non-volatile or volatile storage or memory. At scan data deriving instruction 250 a user employs the scanner 150 to scan a patient's particular identifier and individual blood unit products. The scanner 150 may be a laser or radio frequency scanner as described previously, or another type of scanner that would be known to one of skill in the art. The scan data that is derived from the scan data deriving instruction 250 is provided to a blood infusion protocol instruction 260. Blood infusion protocol instruction 260 may be run by a processor such as an Intel or AMD processor, and may be one of a plurality of blood infusion protocols where a user has selected the particular protocol to run.

Blood infusion protocol instruction 260 is configured to provide data to a data display instruction 270 and to an instruction for both receiving and processing scan data 280. Data display instruction 270 may be instructions embedded in a video display circuit board, and is configured to receive data and to process the data for presentation to a display unit, for example, display unit 300.

Receiving and processing scan data instruction 280 receives data from blood infusion protocol instruction 260, and is configured to evaluate the scan data derived from the patient's particular identifier and individual blood unit products. If, on an initial verification scan, the patient identifier matches an individual blood unit, then an instruction is provided to data display instruction 270 enabling a display of the screenshot of the individual blood unit to change to a 'VERIFIED' status with a change in fill (or background) color, for instance, to a red background and with the word 'VERIFIED,' for example, as shown by Figure 15. The same instruction process is provided for each individual blood product that matches the patient identifier. Once an individual blood unit has been provided with a 'VERIFIED' status, a further instruction must be received at receiving and processing scan data instruction 280 noting that a second verification scan has been performed. If both the first and the second verification scans match both the individual blood product and the patient identifier, then an individual blood unit may be designated as infusion eligible, with the user provided the ability to note the individual blood product as 'TRANSFUSING' as noted by an instruction from receiving and processing scan data instruction 280 to display instruction 270, and as ultimately displayed on display unit 300.

If a patient identifier fails to match an individual blood unit identifier in the scan data provided to receiving and processing instruction 280, then an abort instruction is generated by mismatch/abort instruction 290. Mismatch/abort instruction 290 provides abort instructions to protocol instruction 260, where it is received and processed. In certain exemplary embodiments, when protocol instruction 260 receives an abort instruction, the entire blood infusion protocol is aborted and data is sent to display instruction 270 for display unit 300 noting that the protocol has been aborted. In certain exemplary embodiments, when an abort instruction is received at protocol instruction 260, only the individual mismatched blood units are noted as aborted on display unit 300, and those blood units are segregated from verified and infusion eligible blood units.

Figure 3 is a flow diagram of an exemplary embodiment illustrating a transfusions verification (TV) process or protocol for blood infusion management in certain care environments, such as an operating room. As provided in the figure, a medical professional scans a patient identifier 101, such as an amband, a bracelet, or an anklet. The scanned data is provided to a running protocol, such as a software program encoded for a graphical user interface, such as a C program or a C++ program. The transfusion verification (TV) process has been previously configured for a determination of the protocols available, for instance whether the available protocols will include a rapid infusion protocol or a standard protocol, or both. At decision block 102, the transfusion verification (TV) process determines if the location is an operating room. If not, then the transfusion verification (TV) system defaults to a standard infusion verification protocol 103.

If yes, then the transfusion verification (TV) process selects a rapid infusion (RI) protocol at block 104 and the rapid infusion (RI) screen is displayed on the display unit 170 with a visual indication, for example, a red border that alerts the user that the rapid infusion (RI) protocol has been instigated. For example, the red border 1401 shown in Figure 14 may be used to provide a visual indication to the user that the rapid infusion (RI) protocol is underway. The rapid infusion (RI) protocol requires an initial verification scan of each individual blood unit. If the individual blood unit matches the patient identifier, it is then provided with a 'VERIFIED' status at block 106 that may be displayed on a display with a change in background color to red and/or the word 'VERIFIED.' Decision block 109 inquires if there are more products to scan, and each blood product is scanned unit each unit is initially verified. If any unit does not match, it is not provided a verified status and is segregated.

In certain exemplary embodiments, an individual blood unit that does not match is not allowed to possess a verified status and also creates both a visual an audible alarm to notify medical personnel that the blood unit is a mismatch. Blood units that do match are provided the option of having infusion documentation completed at block 107. Infusion documentation may include patient vital signs, witness verification, an infusion checklist, and blood volume. Either at the end of block 109 (asking if there are more blood products to scan) or block 107 (asking if infusion documentation is to be completed at that time), the user is provided with decision block 108, that inquires if transfusion is to take place at that moment If not, the blood products are returned to storage, such as a cooler or refrigerator 110, where they await a call from a medical professional 111, such as an anesthesiologist calling for a blood infusion on a patient.

At activity block 112, the individual blood products with a 'VERIFIED' status must once again be scanned to determine that they do, indeed, match the previously scanned patient identifier. If any blood product does not match, it is not given a 'TRANSFUSING status and loses its 'VERIFIED' status, and is segregated. In certain exemplary embodiments, any individual blood unit that does not match creates both a visual and an audible alarm to notify medical personnel that the blood unit is a mismatch. In certain exemplary embodiments, when any of the two verification scans do not match the patient identifier, the blood infusion protocol is aborted. In certain exemplary embodiments, when any of the two verification scans do not match the patient identifier, the display is provided with a protocol abortion as to the individual blood unit that does not match. In certain exemplary embodiments, when an individual blood unit does not match the patient identifier, the blood infusion protocol is allowed to continue with compliant, matching individual blood units, but the non-matching blood unit status is reverted to non-verified and the screenshot for the non-matching individual blood unit loses its red fill, or red background, and the user is provided with the option of returning to the point of having to perform two verification scans in order to reach a 'TRANSFUSING' status for that individual blood unit.

After the second verification scan where the blood products match the patient identifier at block 112, the matching blood products are given a 'TRANSFUSING' status, and the patient's needs are met with compliant blood products. After the patient's needs are met, activity block 113 allows for the patient record to proceed with the process of completion, or with closing the individual record. Decision block 114 inquires if the transfusion is complete. If no, the patient record may be temporarily exited at function 116.

Ultimately, when the transfusion is complete, at activity block 114, the medical professional is given the option of performing a second scan of the patient's identifier to bring all previously 'TRANSFUSING' individual blood units up-to-date with a 'COMPLETE' status for the blood unit in question. Alternatively, the medical professional may scan each individual blood unit a third time to individually bring each individual blood unit up-to-date with a 'COMPLETE' status. Once all individual blood units have attained a 'COMPLETE' status, the patient record may be exited at function block 117.

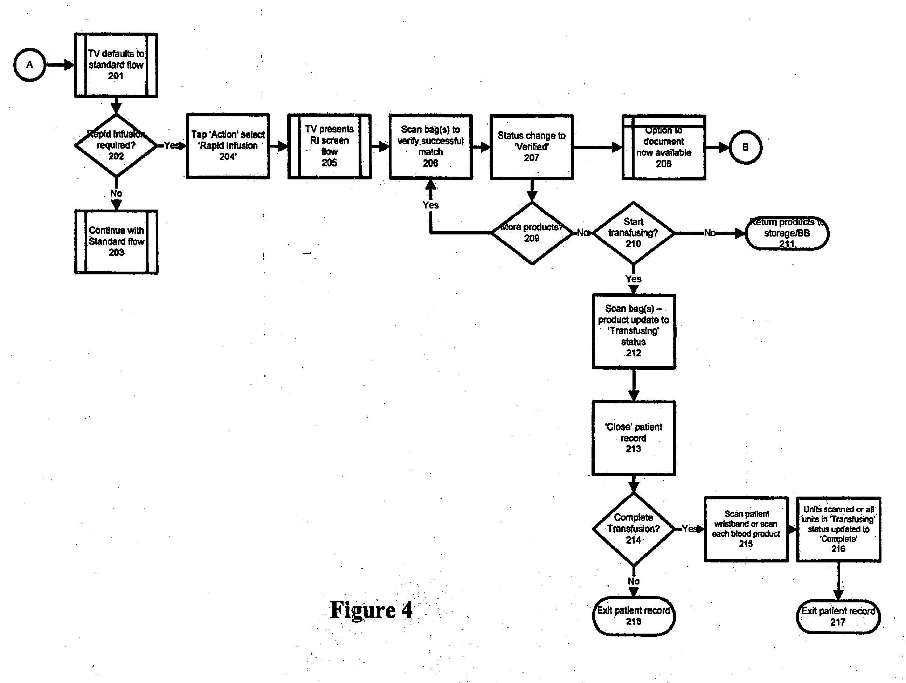

Figure 4 is a flow diagram of an exemplary embodiment illustrating a transfusion verification (TV) process or protocol for blood infusion management in certain care environments, such as an emergent situation. As provided in the figure, because the location includes emergent situations, the transfusion verification (TV) process defaults to an initial query asking the user if the present procedure requires rapid infusion. If the user determines that rapid infusion (RI) is needed, the user selects same at block 204. The transfusion verification (TV) process then begins the rapid infusion protocol and presents a visual indicator on the display 170 indicating, for example, that the rapid infusion protocol has been instigated. An example of a visual indicator would be the red border 1401 shown in Figure 14, for instance.

At a point in time prior to block 206, a medical professional is required to scan a patient identifier, such as an armband, a bracelet, or an anklet. The scanned data is provided to the rapid infusion (RI) protocol. The rapid infusion protocol may be configured in a software program encoded for a graphical user interface, such as a C program or a C++ program. The transfusion verification (TV) process has been previously configured for a determination of the protocols available, for instance whether the available protocols will include a rapid infusion protocol or a standard protocol, or both. At decision block 202, the transfusion verification (TV) process determines if a rapid infusion (RI) protocol has been configured for that location. If not, then the system defaults to a standard infusion verification protocol 203.

If yes, then a rapid infusion (RI) protocol is allowed for user selection at block 104. The rapid infusion (RI) protocol screen includes a brightly colored border in all screenshots, for instance, as shown in Figure 14 as element 1401, to notify a user that the rapid infusion protocol has been selected and is being processed, as noted by block 205. The rapid infusion (RI) protocol requires an initial verification scan of each individual blood unit at activity block 206. If the individual blood unit matches the patient identifier, it is then provided with a 'VERIFIED' status at block 207 that may be displayed on a display (for example, display 170 that is referenced in relation to Figure 1) with a change in background color to red and/or the word 'VERIFIED.' Decision block 209 inquires if there are more products to scan, and each blood product is scanned until each blood product unit is initially verified. If any blood product unit does not match, it is not provided with a verified status, and is segregated.

In certain exemplary embodiments, an individual blood unit that does not match is not allowed to possess a verified status and also creates both a visual and an audible alarm to notify medical personnel that the blood unit is a mismatch. Blood units that do match are provided the option of having infusion documentation completed at block 208. Infusion documentation may include patient vital signs, witness verification, an infusion checklist, and blood volume. Either at the end of block 209 (asking if there are more blood products to scan) or block 207 (asking if infusion documentation is to be completed at that time), the user is provided with decision block 210, that inquires if transfusion is to take place at that moment. If not, the blood products are returned to storage, such as a cooler or refrigerator 211.

If transfusion is to take place, activity block 212 requires that each individual blood products with a 'VERIFIED' status be scanned once again to determine that they do, in fact, match the previously scanned patient identifier. If any blood product does not match, it is not given a 'TRANSFUSING' status and loses its 'VERIFIED' status, and is segregated. In certain exemplary embodiments, any individual blood unit that does not match creates both a visual and an audible alarm to notify medical personnel that the blood unit is a mismatch. In certain exemplary embodiments, when any of the two verification scans do not match the patient identifier, the blood infusion protocol is aborted. In certain exemplary embodiments, when any of the two verification scans do not match the patient identifier, the display is provided with a protocol abortion as to the individual blood unit that does not match. In certain exemplary embodiments, when an individual blood unit does not match the patient identifier, the blood infusion protocol is allowed to continue with compliant, matching individual blood units, but the non-matching blood unit status is reverted to a non-verified status and the screenshot for the non-matching individual blood unit loses its red fill, or red background, and the user is provided with the option of returning to the point of having to perform two verification scans in order to reach a 'TRANSFUSING' status for that individual blood unit.

After the second verification scan where the blood products match the patient identifier at block 212, the matching blood products are given a 'TRANSFUSING' status, and the patient's needs are met with compliant blood products. After the patient's needs are met, activity block 213 allows for the patient record to proceed with the process of completion, or with closing the individual record. Decision block 214 inquires if the transfusion is complete. If no, the patient record may be temporarily exited at function 218.

Ultimately, when the transfusion is complete, at activity block 215, the medical professional is given the option of performing a second scan of the patient's identifier to bring all previously 'TRANSFUSING' individual blood units up-to-date with a 'COMPLETE' status for the blood unit in question. Alternatively, the medical professional may scan each individual blood unit a third time to individually bring each individual blood unit up-to-date with a 'COMPLETE' status at block 216. Once all individual blood units have attained a 'COMPLETE' status, the patient record may be exited at function block 217.

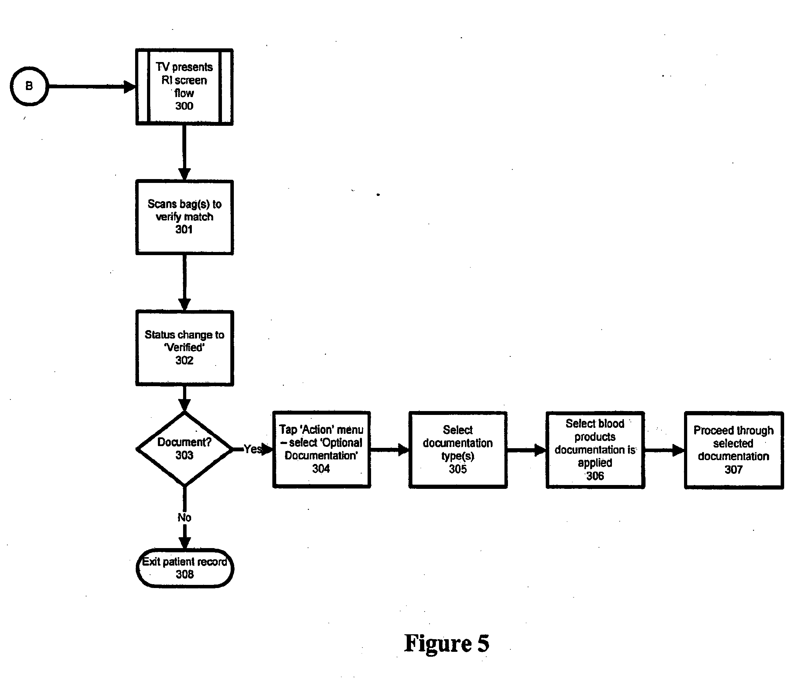

Figure 5 is a is a flow diagram of aspects of exemplary embodiments illustrating a documentation process that may be provided to medical professional users at the end of the blood transfusion processes described herein, for example, for those processes described in relation to Figures 3 and 4. At activity block 300, the transfusion verification process has previously presented a blood infusion protocol, for instance a rapid infusion protocol as described in relation to Figures 3 and 4.

In certain exemplary embodiments, documentation of the infusion process may be completed either after a first verification scan, or upon an individual blood unit attaining a complete status, as described above. Figure 5 reflects an instance of completing infusion documentation after blood product units have attained an initial 'VERIFIED' status. At block 300 the transfusion verification (TV) process defaults to a rapid infusion (RI) screen as a result of pre-configuration. At block 301, individual blood units undergo an initial verification scan. If the blood unit(s) match the particular patient identifier, then the individual blood unit's status is changed to 'VERIFIED' at block 302.

Decision block 303 then inquires if the user sees the present moment as an opportune time to perform infusion documentation. If not, then the option is provided to exit the patient record at function 308, or to continue with an infusion process without performing infusion documentation at that moment. If the user selects 'yes' from block 303, optional documentation may be selected from block 304.

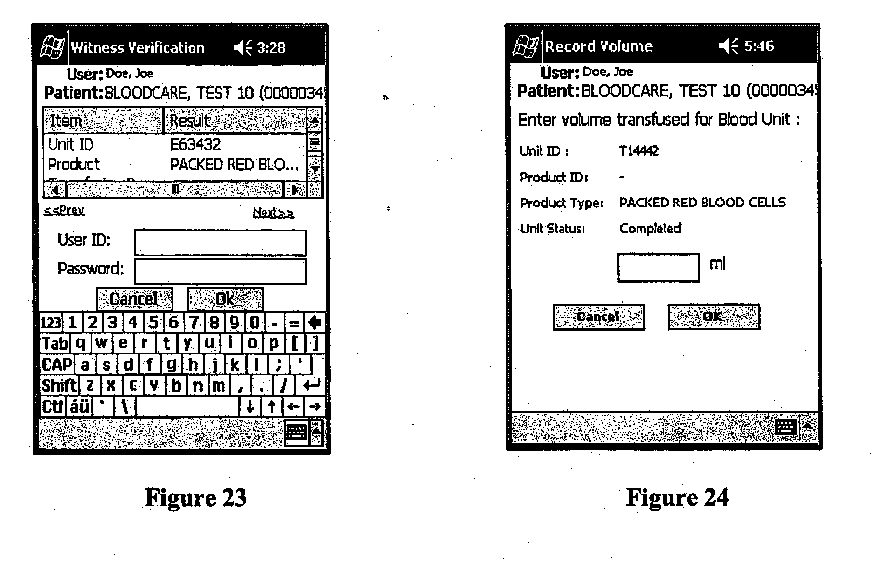

In certain exemplary embodiments, infusion documentation may include patient vital signs, witness verification, an infusion checklist, and blood volume,. The user is provided with the option of selecting what documentation type(s) is/are desired at block 305, for example, as shown in Figure 22. In block 306, the user is provided the option of selecting the blood product types for which infusion documentation is to be performed, for example, a choice may be presented between packed red blood cells and platelets, or other blood products, as shown in Figure 21. At block 307, various entry screens are provided to the user, for example, as shown in Figures 23 and 24, for entering the necessary information into the selected documents for the selected blood types, at the end of which, the infusion documentation is complete.

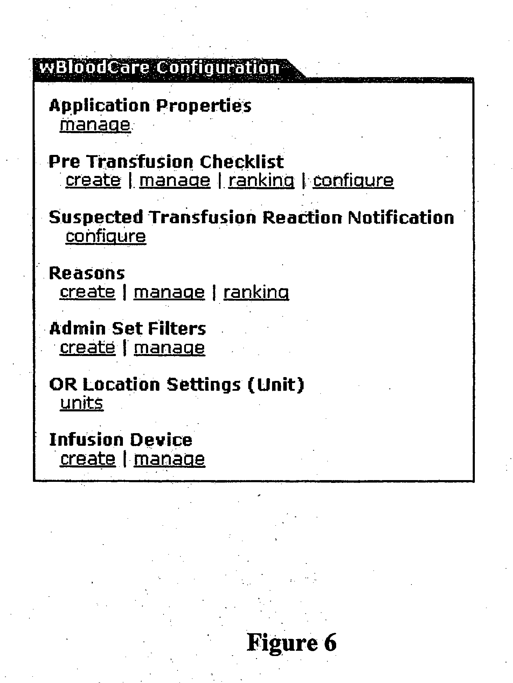

Figure 6 is a screenshot of an exemplary embodiment showing configuration options including application properties, a pre-transfusion checklist, a reaction notification setting, administrative filters, location settings, and type of infusion device.

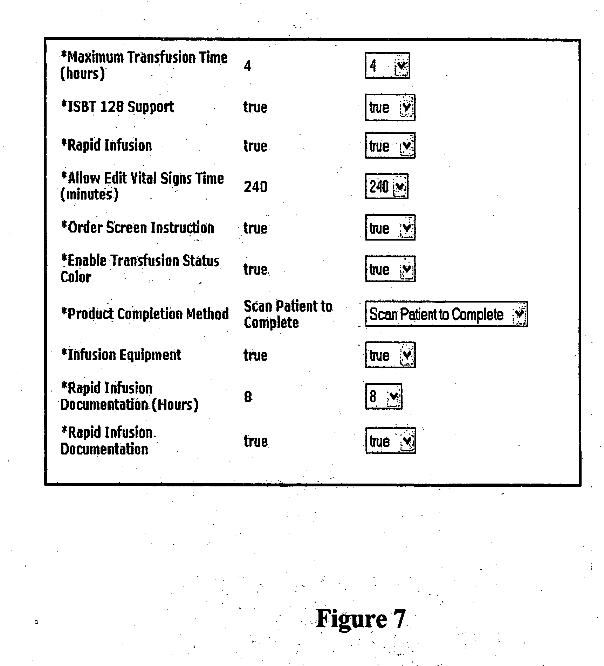

Figure 7 is a screenshot of an exemplary embodiment showing additional configuration options including maximum time settings allowed for infusion, ISBT 128 support, whether the location will allow rapid infusion, the length of time that vital signs may be edited, whether the order screen will be available, visual color alert selection, selection for completion method (for instance, whether by scanning the patient identifier or by scanning every used blood product unit), a selection for blood infusion equipment type or presence, the time frame in which infusion documentation must be completed, and whether infusion documentation will be needed.

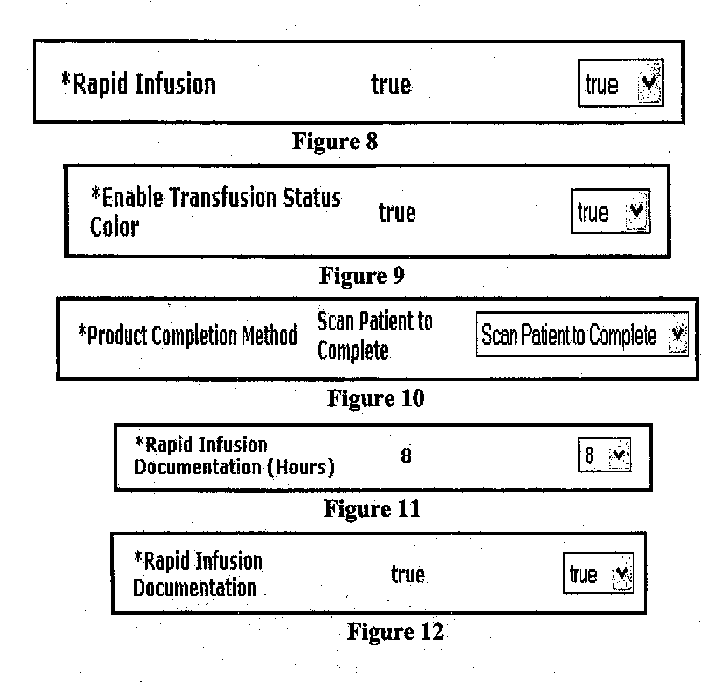

Figure 8 is a screenshot of an exemplary embodiment showing a toggle button or selection for whether or not a rapid infusion protocol may be implemented.

Figure 9 is a screenshot of an exemplary embodiment showing a toggle button or selection for whether or not a transfusion status color will change once a transfusion begins.

Figure 10 is a screenshot of an exemplary embodiment showing a toggle button or selection for the type of completion process that will be followed, for instance, whether a single scan of a patient identifier will complete the process for all used blood product units, or whether a scan of each individual used blood product unit will be required to complete the process.

Figure 11 is a screenshot of an exemplary embodiment showing a toggle button or selection for the time frame in which infusion documentation must be completed. This option may be configured for up to a 24 hour setting, but typically will be set at 4 to 12 hours.

Figure 12 is a screenshot of an exemplary embodiment showing a toggle button or selection for whether or not infusion documentation will be provided.

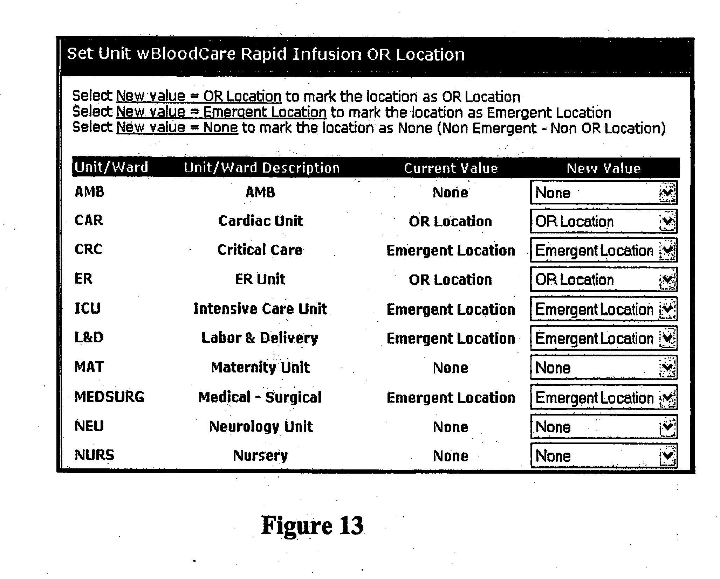

Figure 13 is a screenshot of an exemplary embodiment showing setup values for different locations within a facility and the type of blood infusion protocol that will be allowed at particular locations. For instance, critical care is marked as an emergent location and will therefore be allowed to perform rapid infusion protocols, while the cardiac unit is an operating room location and will therefore be allowed to perform operating room protocols.

Figure 14 is a screenshot of an exemplary embodiment wherein the border of the screenshot has been colored a bright red, for instance as shown by element 1401 in the figure, indicating that the protocol underway is a rapid infusion protocol.

Figure 15 is a screenshot of an exemplary embodiment showing that once a blood product unit has been given a status 'TRANSFUSING' the status may be intentionally reverted back to `VERIFIED.'

Figures 16 and 17 are screenshots of an exemplary embodiment showing that once a blood infusion is complete, a user may begin the process of documenting that the used blood products have a 'COMPLETE' status. The same screen may be displayed if a user chooses to exit the application or close a patient's record after accessing the rapid infusion order screen and there exists blood unit(s) with a status of 'TRANSFUSING.' If so, the application will prompt the user to complete the transfusion. As shown in Figures 16 and 17, the user is being asked if they wish to complete the process by indicating that the transfusing status is complete for all blood product units. The manner of reaching a 'COMPLETE' status is configured at the configuration screen, for instance as shown in relation to Figures 7 and 10, and may be set up to allow a 'COMPLETE' status to be achieved by a singular scan of a patient identifier. Alternatively, the system may be set up to allow a 'COMPLETE' status to be achieved by a scan of each used blood product unit.

Figure 18 is a screenshot of an exemplary embodiment showing that a blood unit is being updated from transfusing to complete by a scan of the blood unit.

Figure 19 is a screenshot of an exemplary embodiment showing that a blood unit is being updated from transfusing to complete by a scan of the blood unit.

Figure 20 is a screenshot of an exemplary embodiment showing that optional documentation may be accessed from an action menu. This provides a user with the ability to choose optional documentation for a rapid infusion work flow.

Figure 21 is a screenshot of an exemplary embodiment showing that a user may select certain blood products for optional documentation. For instance, as shown in the figure, packed red blood cells may be selected for documentation.

Figure 22 is a screenshot of an exemplary embodiment showing that a user may select certain documentation for the previously selected blood type, for instance that blood product type selected in reference to Figure 21. As shown in Figure 22, the user may select a checklist, vital signs, witness verification, and/or volume infused. A user may also simply selection 'ALL DOCS' and thereby complete documentation for all document types displayed.

Figure 23 is a screenshot of an exemplary embodiment showing that a user may select witness verification documentation. The screen includes the hyperlinks of <<Prev and Next>>, which allow a user to toggle through different blood product units for witness verification of each blood product unit previously selected.

Figure 24 is a screenshot of an exemplary embodiment showing that a user may record the volume of blood products infused into the patient. When multiple blood product units are selected for documentation the volume infused screen for each product is selected.

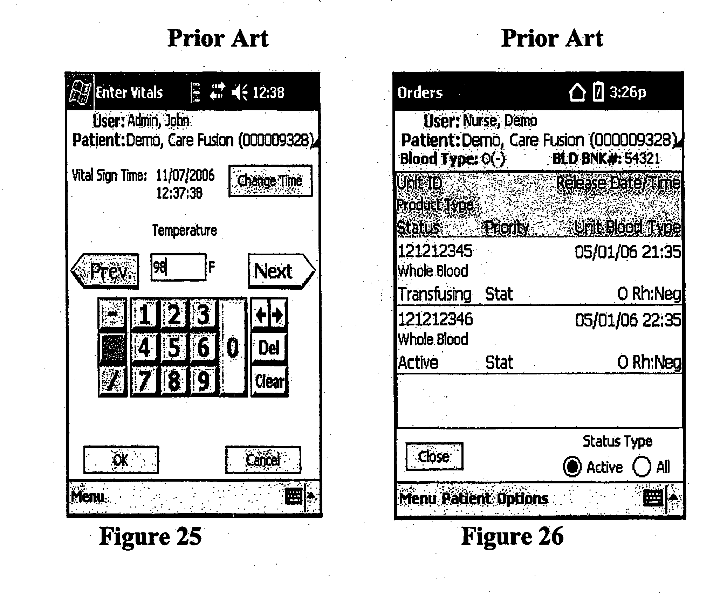

Figures 25 and 26 are screenshots of prior art blood infusion protocol processes showing an order screen for a standard infusion process where a user is required to enter vital signs prior to going to a blood bank to pick up needed blood products. Once the vital signs have been recorded in Figure 25, then the user is allowed to scan individual blood products for processing in Figure 26.

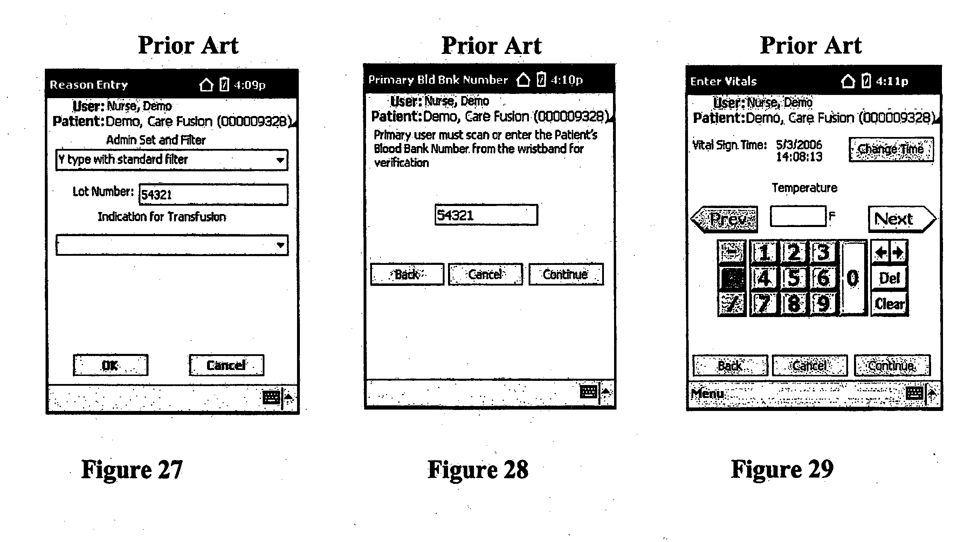

Figures 27 - 29 are screenshots of prior art blood infusion protocol processes showing screens for set and filter and indication for transfusion, primary blood bank number, and entry of vital signs, respectively.

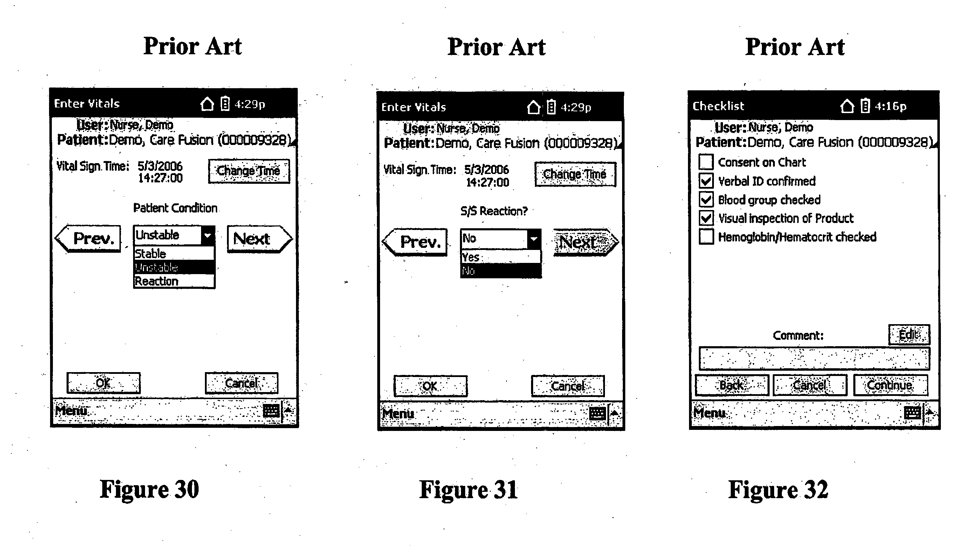

Figures 30 - 32 are screenshots of prior art blood infusion protocol processes showing screens for documenting patient condition, entry of vital signs, and a blood infusion checklist, respectively.

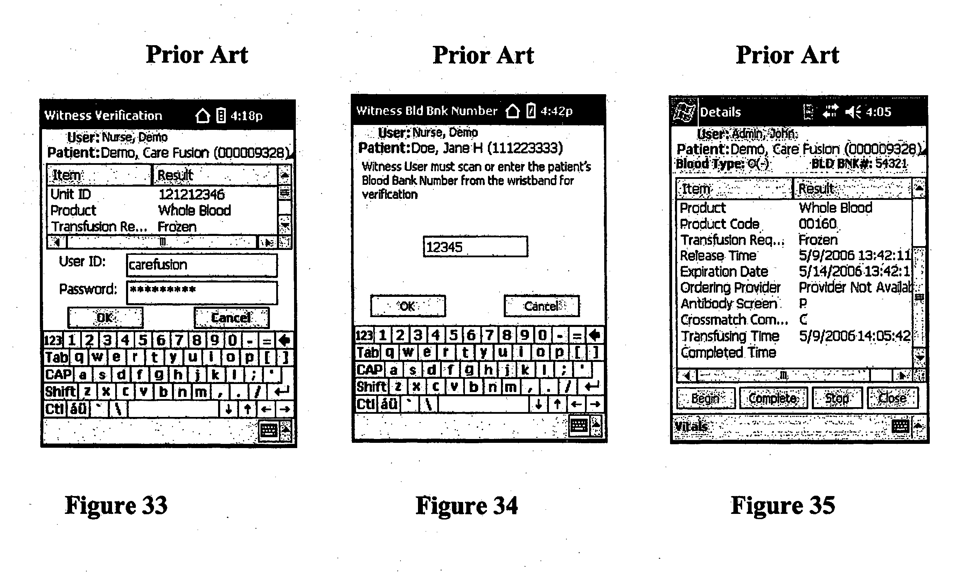

Figures 33 - 35 are screenshots of prior art blood infusion protocol processes showing screens for witness verification, scan blood bank number, and review/detail information, respectively.

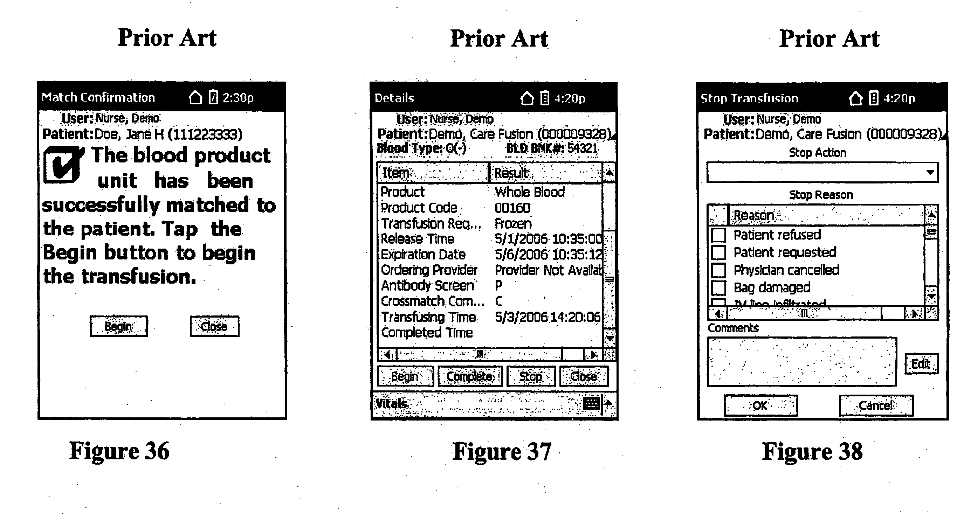

Figures 36 - 38 are screenshots of prior art blood infusion protocol processes showing screens for starting a transfusion process, detailing the transfusion process, and stopping the transfusion process, respectively.

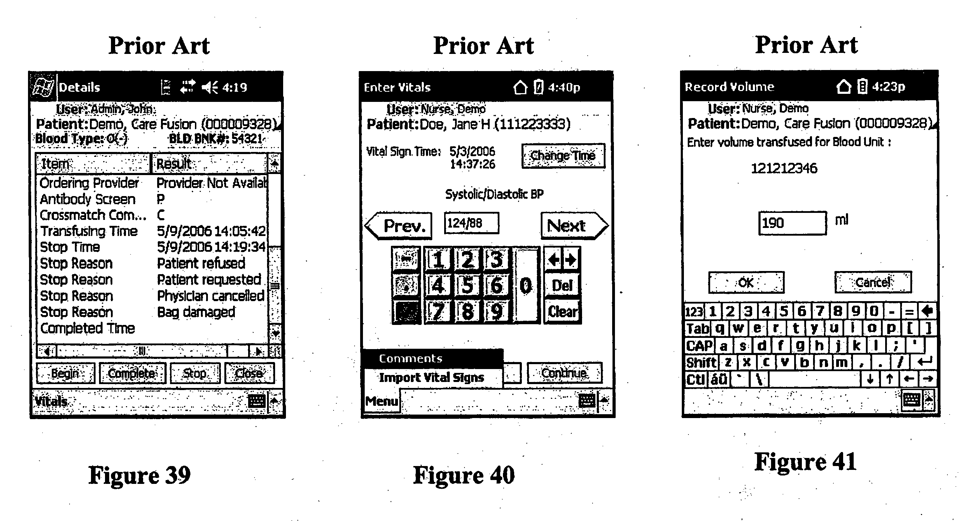

Figures 39 - 41 are screenshots of prior art blood infusion protocol processes showing screens for completing a blood infusion process including details, entering vital signs, and recording volume infused, respectively.

As described above, it is possible to implement some aspects of the present inventions as a method and/or in a computer system. The computer system may include a bus or other communication mechanism for communicating information, and a processor coupled with the bus for processing information. The computer system may also include a memory coupled to the bus for storing information and instructions to be executed by the processor. The memory may also be used for storing temporary variables or other intermediate information during execution of instructions by the processor. The computer system further may also include a data storage device, such as a magnetic disk or optical disk, coupled to the bus for storing information and instructions. The computer system may be coupled to a display device for displaying information to a user. An input device, such as, for example, a keyboard or a mousse may also be coupled to the computer system for communicating information and command selections to the processor.

According to certain exemplary embodiments, protocol selection and execution may be performed utilizing software, an algorithm, a processor, and/or a computer system in response to an output of a processor executing one or more sequences of one or more instructions contained in a memory. Such instructions may be read into the memory from a machine-readable medium, such as a data storage device.

The description of the invention is provided to enable any person skilled in the art to practice the various embodiments described herein. While the present invention has been particularly described with reference to the various figures and embodiments, it should be understood that these are for illustration purposes only and should not be taken as limiting the scope of the inventions.

It is understood that the specific order or hierarchy or steps in the processes disclosed herein is an illustration of exemplary approaches.

While certain aspects and embodiments of the inventions have been described, these have been presented by way of example only, and are not intended to limit the scope of the inventions. The accompanying claims and their equivalents are intended to cover such forms or modifications as would fall within the scope and spirit of the inventions.

## Claims

1. A blood infusion management system, comprising:
means (150) for deriving scan data from individual blood units and a patient identifier particular to an individual patient;
means (170) for visually displaying on a display at least one individual blood unit status within a blood infusion protocol;
means (160) for running the blood infusion protocol;
means (160) for providing data to the display for visual display of the blood infusion protocol, including the at least one individual blood unit status;
means (160) for receiving and processing the scan data from the scanner; and
means (160) for aborting the blood infusion protocol if the processed scan data indicates that any of the individual blood units do not match the patient identifier,
wherein the blood infusion protocol is configured to require at least two verification scans of an individual blood unit where the individual blood unit must match the patient identifier to acquire a transfusing status on the display for the individual blood unit.

2. The blood infusion management system of claim 1, further comprising:
means (165) for selecting a level of blood infusion service based on at least one of a service level setting and a user selection, wherein the blood infusion protocol is determined by the selected level of blood infusion service and is selected from a plurality of blood infusion protocols, the blood infusion protocol is configured to require a scan of one of the patient identifier or a used individual blood unit to acquire a complete status on the display for the used individual blood unit, and the blood infusion protocol is configured to provide user access to infusion documentation after acquiring a complete status.

3. The blood infusion management system of claim 1, further comprising: means (160) for changing the status of the individual blood unit to a verified status on the display if the processed scan data indicates that the individual blood unit matches the patient identifier after a first verification scan, as noted by at least one of a color change and a message on the display.

4. The blood infusion management system of claim 2, wherein the infusion documentation comprises of at least one of patient vital signs, a checklist, a witness verification, and blood unit volume.

5. The blood infusion management system of claim 2, further comprising: means (160) for providing a time frame in which the infusion documentation must be completed.

6. The blood infusion management system of claims 1 to 5, wherein:
the means for deriving scan data comprise a scanner (150) configured to derive scan data from individual blood units and a patient identifier particular to an individual patient;
the means for visually displaying comprise a display (170) configured to visually display at least one individual blood unit status within a blood infusion protocol; and
the means for running the blood infusion protocol, means for providing data to the display, means for receiving and processing the scan data from the scanner, and means for aborting the blood infusion protocol comprise a processor (160) configured to run the blood infusion protocol, to provide data to the display for visual display of the blood infusion protocol, including the at least one individual blood unit status, to receive and process the scan data from the scanner, and to abort the blood infusion protocol if the processed scan data indicates that any of the individual blood units do not match the patient identifier.

7. A method for authorizing individual blood product units for infusion to a patient and for providing information on the status of the individual blood product unit within a blood infusion protocol the method comprising:
deriving scan data from individual blood units (105) and a patient identifier particular to an individual patient (101);
evaluating (106) whether the scan data consists of a match between the patient identifier and each individual blood unit that is intended to be used for the patient for the instant infusion;
conducting a second verification scan (112) of each individual blood unit, where each individual blood unit must once again match the initial scan of the patient identifier prior to allowing infusion of the blood product.

8. The method of claim 7, further comprising:
selecting a level of blood infusion service based on at least one of a service level setting and a user selection,
wherein the selected level of blood infusion service determines a blood infusion protocol selected from a plurality of blood infusion protocols, the blood infusion protocol being configured to require a scan of one of the patient identifier or a used individual blood unit to acquire a complete status on the display for the used individual blood unit, and the blood infusion protocol being configured to provide user access to infusion documentation after acquiring a complete status.

9. The method of claim 7, further comprising changing (106) the status of the individual blood unit to a verified status on a display if the processed scan data indicates that the individual blood unit matches the patient identifier after a first verification scan, as noted by at least one of a color change and a message on the display.

10. The method of claim 8, wherein the infusion documentation comprises at least one of patient vital signs, a checklist, a witness verification, and blood unit volume.

11. The method of claim 8, further comprising providing a time frame in which the infusion documentation must be completed.

12. A computer-readable medium having computer-executable instructions for causing a processor to execute instructions for a blood infusion protocol by performing steps comprising:
deriving (250) scan data from individual blood units and a patient identifier particular to an individual patient;
visually displaying (270) on a display (300) at least one individual blood unit status within a blood infusion protocol;
running (260) the blood infusion protocol;
providing data (270) to the display for visual display of the blood infusion protocol, including the at least one individual blood unit status;
receiving and processing (280) the scan data from the scanner; and aborting (290) the blood infusion protocol if the processed scan data indicates that any of the individual blood units do not match the patient identifier,
wherein the blood infusion protocol is configured to require at least two verification scans of an individual blood unit, where the individual blood unit must match the patient identifier to acquire a transfusing status on the display for the individual blood unit.

13. The computer-readable medium of claim 12, the steps further comprising:
selecting a level of blood infusion service based on at least one of a service level setting and a user selection,
wherein the blood infusion protocol is determined by the selected level of blood infusion service and is selected from a plurality of blood infusion protocols, the blood infusion protocol is configured to require a scan of one of the patient identifier or a used individual blood unit to acquire a complete status on the display for the used individual blood unit, and the blood infusion protocol is configured to provide user access to infusion documentation after acquiring a complete status.

14. The computer-readable medium of claim 12, the steps further comprising: changing the status of the individual blood unit to a verified status on the display when the processed scan data indicates that the individual blood unit matches the patient identifier after a first verification scan, as noted by at least one of a color change and a message on the display.

15. The computer-readable medium of claim 14, wherein the infusion documentation comprises of at least one of patient vital signs, a checklist, a witness verification, and blood unit volume; the steps further comprising: requiring a time frame in which the infusion documentation must be completed.

## Patentansprüche

1. Blutinfusionsmanagementsystem, das folgendes umfasst:
Mittel (150) zum Ableiten von Scandaten von einzelnen Bluteinheiten und einer für einen individuellen Patienten bestimmten Patientenkennung;
Mittel (170) zum visuellen Anzeigen mindestens eines individuellen Bluteinheitsstatus in einem Blutinfusionsprotokoll auf einer Anzeige;
Mittel (160) zum Ausführen des Blutinfusionsprotokolls;
Mittel (160) zum Bereitstellen von Daten an die Anzeige zur visuellen Anzeige des Blutinfusionsprotokolls, einschließlich des mindestens einen individuellen Bluteinheitsstatus;
Mittel (160) zum Empfangen und Verarbeiten der Scandaten von dem Scanner; und
Mittel (160) zum Abbrechen des Blutinfusinnsprotakolls, wenn die verarbeiteten Scandaten anzeigen, dass beliebige der individuellen Bluteinheiten nicht mit der Patientenkennung übereinstimmen;
wobei das Blutinfusionsprotokoll so konfiguriert ist, dass es mindestens zwei Verifizierungsscans einer individuellen Bluteinheit erfordert, wobei die individuelle Bluteinheit mit der Patientenkennung übereinstimmen muss, um einen Transfusionsstatus auf der Anzeige für die individuelle Bluteinheit zu erfassen.

2. Blutinfusionsmanagementsystem nach Anspruch 1, wobei dieses ferner folgendes umfasst:
Mittel (165) zur Auswahl einer Stufe einer Blutinfusionsleistung auf der Basis mindestens einer Leistungsstufeneinstellung und einer Benutzerauswahl, wobei das Blutinfusionsprotokoll bestimmt ist durch die ausgewählte Stufe der Blutinfusionsleistung und ausgewählt wird aus einer Mehrzahl von Blutinfusionsprotokollen, wobei das Blutinfusionsprotokoll so konfiguriert ist, dass es einen Scan der Patientenkennung oder einer verwendeten individuellen Bluteinheit erfordert, um einen vollständigen Status auf der Anzeige für die verwendete individuelle Bluteinheit erfordert, und wobei das Blutinfusionsprotokoll so konfiguriert ist, dass es Benutzerzugriff auf die Infusionsdokumentation nach dem Erfassen eines vollständigen Status bereitstellt.

3. Blutinfusionsmanagementsystem nach Anspruch 1, wobei dieses ferner folgendes umfasst: Mittel (160) zum Verändern des Status der individuellen Bluteinheit in einen verifizierten Status auf der Anzeige, wenn die verarbeiteten Scandaten anzeigen, dass die individuelle Bluteinheit nach einem ersten Verifizierungsscan mit der Patientenkennung übereinstimmt, was durch mindestens eine Farbveränderung oder eine Nachricht auf der Anzeige angezeigt wird.

4. Blutinfusionsmanagementsystem nach Anspruch 2, wobei die Infusionsdokumentation mindestens eines der folgenden umfasst:
Patientenvitalparameter, eine Checkliste, eine Zeugenverifizierung oder ein Bluteinheitsvolumen.

5. Blutinfusionsmanagementsystem nach Anspruch 2, wobei dieses ferner folgendes umfasst: Mittel (160) zum Bereitstellen eines Zeitrahmens, innerhalb welchem die Infusionsdokumentation abgeschlossen sein muss.

6. Blutinfusionsmanagementsystem nach einem der Ansprüche 1 bis 5, wobei:
die Mittel zum Ableiten von Scandaten einen Scanner (150) umfassen, der so konfiguriert ist, dass er Scandaten von einzelnen Bluteinheiten und einer für einen individuellen Patienten bestimmten Patientenkennung ableitet;
die Mittel zum visuellen Anzeigen eine Anzeige (170) umfassen, die so konfiguriert ist, dass sie mindestens einen individuellen Bluteinheitsstatus in einem Blutinfusionsprotokoll visuell anzeigt; und
die Mittel zum Ausführen des Blutinfusionsprotokolls, die Mittel zum Bereitstellen von Daten an die Anzeige, die Mittel zum Empfangen und Verarbeiten der Scandaten von dem Scanner und die Mittel zum Abbrechen des Blutinfusionsprotokolls einen Prozessor (160) umfassen, der so konfiguriert ist, dass er das Blutinfusionsprotokoll ausführt, Daten an die Anzeige zur visuellen Anzeige des Blutinfusionsprotokolls bereitstellt, einschließlich des mindestens einen individuellen Bluteinheitsstatus, die Scandaten von dem Scanner empfängt und verarbeitet und das Blutinfusionsprotokoll abbricht, wenn die verarbeiteten Scandaten anzeigen, dass eine der individuellen Bluteinheiten nicht mit der Patientenkennung übereinstimmt.

7. Verfahren zum Autorisieren individueller Blutprodukteinheiten für eine Infusion bei einem Patienten und zum Bereitstellen von Informationen zum Status der individuellen Blutprodukteinheit in einem Blutinfusionsprotokoll, wobei das Verfahren folgendes umfasst:
Ableiten von Scandaten von individuellen Bluteinheiten (105) und einer für einen individuellen Patienten (101) bestimmten Patientenkennung;
Evaluieren (106), ob die Scandaten eine Übereinstimmung zwischen der Patientenkennung und allen individuellen Bluteinheit aufweisen, die für den Patienten zur unmittelbaren Infusion vorgesehen sind;
Durchführen eines zweiten Verifizierungsscans (112) jeder individuellen Bluteinheit, wobei jede individuelle Bluteinheit wiederum mit dem ersten Scan der Patientenkennung übereinstimmen muss, bevor die Infusion des Blutprodukts zugelassen wird.

8. Verfahren nach Anspruch 7, wobei dieses ferner folgendes umfasst:
Auswählen einer Stufe der Blutinfusionsleistung auf der Basis zumindest einer Leistungsstufeneinstellung oder einer Benutzerauswahl;
wobei die ausgewählte Stufe der Blutinfusionsleistung ein Blutinfusionsprotokoll bestimmt, das aus einer Mehrzahl von Blutinfusionsprotokollen ausgewählt ist, wobei das Blutinfusionsprotokoll so konfiguriert ist, dass es einen Scan der Patientenkennung oder einer verwendeten individuellen Bluteinheit benötigt, um einen vollständigen Status auf der Anzeige für die verwendete individuelle Bluteinheit zu erfassen, und wobei das Blutinfusionsprotokoll so konfiguriert ist, dass es nach der Erfassung eines vollständigen Status Benutzerzugriff auf die Infusionsdokumentation bereitstellt.

9. Verfahren nach Anspruch 7, wobei dieses ferner das Verändern (106) des Status der individuellen Bluteinheit in einen verifizierten Status auf einer Anzeige umfasst, wenn die verarbeiteten Scandaten anzeigen, dass die individuelle Bluteinheit nach einem ersten Verifizierungsscan mit der Patientenkennung übereinstimmt, gemäß der Anzeige durch wenigstens eine Farbveränderung oder eine Nachricht auf der Anzeige.

10. Verfahren nach Anspruch 9, wobei die Infusionsdokumentation mindestens eines der folgenden umfasst: Patientenvitalparameter, eine Checkliste, eine Zeugenverifizierung oder ein Bluteinheitsvolumen.

11. Verfahren nach Anspruch 8, wobei dieses ferner das Bereitstellen eines Zeitrahmens umfasst, innerhalb dem die Infusionsdokumentation abgeschlossen sein muss.

12. Computerlesbares Medium mit mittels Computer ausführbaren Anweisungen, um zu bewirken, dass ein Prozessor Anweisungen für ein Blutinfusionsprotokoll ausführt, indem Schritte ausgeführt werden, die folgendes umfassen:
Ableiten (250) von Scandaten von individuellen Bluteinheiten und einer für einen individuellen Patienten bestimmten Patientenkennung;
visuelles Anzeigen (270) auf einer Anzeige (300) mindestens eines individuellen Bluteinheitsstatus in einem Blutinfusionsprotokoll;
Ausführen (260) des Blutinfusionsprotokolls;
Bereitstellen von Daten (270) an die Anzeige zur visuellen Anzeige des Blutinfusionsprotokolls, einschließlich des mindestens einen individuellen Bluteinheitsstatus;
Empfangen und verarbeiten (280) der Scandaten von dem Scanner; und Abbrechen (290) des Blutinfusionsprotokolls, wenn die verarbeiteten Scandaten anzeigen, dass beliebige der Bluteinheiten nicht mit der Patientenkennung übereinstimmen;
wobei das Blutinfusionsprotokoll so konfiguriert ist, dass es mindestens zwei Verifizierungsscans einer individuellen Bluteinheit erfordert, wobei die individuelle Bluteinheit mit der Patientenkennung übereinstimmen muss, um einen Transfusionsstatus auf der Anzeige für die individuelle Bluteinheit zu erfassen.

13. Computerlesbares Medium nach Anspruch 12, wobei die Schritte ferner folgendes umfassen:
Auswählen einer Stufe der Blutinfusionsleistung auf der Basis zumindest einer Leistungsstufeneinstellung oder einer Benutzerauswahl;
wobei das Blutinfusionsprotokoll bestimmt wird durch die ausgewählte Stufe der Blutinfusionsleistung ein Blutinfusionsprotokoll und ausgewählt wird aus einer Mehrzahl von Blutinfusionsprotokollen, wobei das Blutinfusionsprotokoll so konfiguriert ist, dass es einen Scan der Patientenkennung oder einer verwendeten individuellen Bluteinheit benötigt, um einen vollständigen Status auf der Anzeige für die verwendete individuelle Bluteinheit zu erfassen, und wobei das Blutinfusionsprotokoll so konfiguriert ist, dass es nach der Erfassung eines vollständigen Status Benutzerzugriff auf die Infusionsdokumentation bereitstellt.

14. Computerlesbares Medium nach Anspruch 12, wobei die Schritte ferner folgendes umfassen: Verändern des Status der individuellen Bluteinheit in einen verifizierten Status auf einer Anzeige, wenn die verarbeiteten Scandaten anzeigen, dass die individuelle Bluteinheit nach einem ersten Verifizierungsscan mit der Patientenkennung übereinstimmt, gemäß der Anzeige durch wenigstens eine Farbveränderung oder eine Nachricht auf der Anzeige.

15. Computerlesbares Medium nach Anspruch 14, wobei die Infusionsdokumentation mindestens eines der folgenden umfasst: Patientenvitalparameter, eine Checkliste, eine Zeugenverifizierung oder ein Bluteinheitsvolumen; wobei die Schritte ferner folgendes umfassen: Erfordern eines Zeitrahmens, innerhalb welchem die Infusionsdokumentation abgeschlossen werden muss.

## Revendications

1. Système de gestion de perfusion de sang, comprenant :
un moyen (150) pour déduire des données de balayage à partir d'unités de sang individuelles et d'un identifiant patient spécifique à un patient individuel ;
un moyen (170) pour afficher visuellement sur un écran d'au moins un statut d'unité de sang individuelle dans un protocole de perfusion de sang;
un moyen (160) pour exécuter le protocole de perfusion de sang ;
un moyen (160) pour fournir des données à l'écran pour l'affichage visuel du protocole de perfusion de sang, y compris l'au moins un statut d'unité de sang individuelle ;
un moyen (160) pour recevoir et traiter les données de balayage provenant du scanneur ; et
un moyen (160) pour interrompre le protocole de perfusion de sang si les données de balayage traitées indiquent que l'une des unités de sang individuelles ne correspond pas à l'identifiant patient,
le protocole de perfusion de sang étant conçu pour exiger au moins deux balayages de vérification d'une unité de sang individuelle, l'unité de sang individuelle devant correspondre à l'identifiant patient pour acquérir un statut de perfusion sur l'écran pour l'unité de sang individuelle.

2. Système de gestion de perfusion de sang selon la revendication 1, comprenant en outre :
un moyen (165) pour sélectionner un niveau de service de perfusion de sang en fonction d'un paramétrage de niveau de service et/ou d'une sélection d'utilisateur, le protocole de perfusion de sang étant déterminé par le niveau sélectionné de service de perfusion de sang et étant choisi parmi une pluralité de protocoles de perfusion de sang, le protocole de perfusion de sang étant conçu pour exiger un balayage de l'identifiant patient et/ou d'une unité de sang individuelle utilisée pour acquérir un statut complet sur l'écran pour l'unité de sang individuelle utilisée, et le protocole de perfusion de sang étant conçu pour fournir un accès utilisateur à la documentation de perfusion après l'acquisition d'un statut complet.

3. Système de gestion de perfusion de sang selon la revendication 1, comprenant en outre : un moyen (160) pour changer le statut de l'unité de sang individuelle en un statut vérifié sur l'écran si les données de balayage traitées indiquent que l'unité de sang individuelle correspond à l'identifiant patient après un premier balayage de vérification, comme indiqué par un changement de couleur et/ou un message sur l'écran.

4. Système de gestion de perfusion de sang selon la revendication 2, la documentation de perfusion comprenant au moins l'un parmi les signes vitaux du patient, une liste de contrôle, une vérification de témoin, et le volume d'unité de sang.

5. Système de gestion de perfusion de sang selon la revendication 2, comprenant en outre : un moyen (160) pour fournir un délai temporel dans lequel la documentation de perfusion doit être complétée.

6. Système de gestion de perfusion de sang selon l'une des revendications 1 à 5 :
le moyen pour déduire des données de balayage comprenant un scanneur (150) conçu pour déduire des données de balayage à partir d'unités de sang individuelles et d'un identifiant patient spécifique à un patient individuel ;
le moyen pour afficher visuellement comprenant un écran (170) conçu pour afficher visuellement au moins un statut d'unité de sang individuelle dans un protocole de perfusion de sang ; et
le moyen pour exécuter le protocole de perfusion de sang, un moyen pour fournir des données à l'écran, un moyen pour recevoir et traiter les données de balayage provenant du scanneur, et un moyen pour interrompre le protocole de perfusion de sang comprenant un processeur (160) conçu pour exécuter le protocole de perfusion de sang, pour fournir des données à l'écran pour l'affichage visuel du protocole de perfusion de sang, y compris l'au moins un statut d'unité de sang individuelle, pour recevoir et traiter les données de balayage provenant du scanneur, et pour interrompre le protocole de perfusion de sang si les données de balayage traitées indiquent que l'une des unités de sang individuelles ne correspond pas à l'identifiant patient.

7. Procédé pour autoriser des unités de produit sanguin individuelles à des fins de perfusion à un patient et pour fournir des informations sur le statut de l'unité de produit sanguin individuelle dans un protocole de perfusion de sang, le procédé comprenant les étapes consistant à :
déduire des données de balayage à partir d'unités de sang individuelles (105) et d'une identifiant patient spécifique à un patient individuel (101) ;
évaluer (106) si les données de balayage sont une correspondance entre l'identifiant patient et chaque unité de sang individuelle qui est destinée à être utilisée pour le patient pour la perfusion immédiate ;
effectuer un second balayage de vérification (112) de chaque unité de sang individuelle, chaque unité de sang individuelle devant correspondre à nouveau au balayage initial de l'identifiant patient avant d'autoriser la perfusion du produit sanguin.

8. Procédé selon la revendication 7, comprenant en outre les étapes consistant à :
sélectionner un niveau de service de perfusion de sang en fonction d'un paramétrage de niveau de service et/ou d'une sélection d'utilisateur,
le niveau sélectionné de service de perfusion de sang déterminant un protocole de perfusion de sang choisi parmi une pluralité de protocoles de perfusion de sang, le protocole de perfusion de sang étant conçu pour exiger un balayage de l'identifiant patient ou/ou d'une unité de sang individuelle utilisée pour acquérir un statut complet sur l'écran pour l'unité de sang individuelle utilisée, et le protocole de perfusion de sang étant conçu pour fournir un accès utilisateur à la documentation de perfusion après l'acquisition d'un statut complet.

9. Procédé selon la revendication 7, comprenant en outre l'étape consistant à changer (106) le statut de l'unité de sang individuelle en un statut vérifié sur un écran si les données de balayage traitées indiquent que l'unité de sang individuelle correspond à l'identifiant patient après un premier balayage de vérification, comme indiqué par un changement de couleur et/ou un message sur l'écran.

10. Procédé selon la revendication 8, la documentation de perfusion comprenant au moins l'un parmi les signes vitaux du patient, une liste de contrôle, une vérification de témoin, et le volume d'unité de sang.

11. Procédé selon la revendication 8, comprenant en outre l'étape consistant à fournir un délai temporel dans lequel la documentation de perfusion doit être complétée.

12. Support lisible par ordinateur ayant des instructions exécutables par ordinateur pour amener un processeur à exécuter des instructions pour un protocole de perfusion de sang en effectuant les étapes consistant à :
déduire (250) des données de balayage à partir d'unités de sang individuelles et d'un identifiant patient spécifique à un patient individuel ;
afficher visuellement (270) sur un écran (300) au moins un statut d'unité de sang individuelle dans un protocole de perfusion de sang ;
exécuter (260) le protocole de perfusion de sang ;
fournir des données (270) sur l'écran pour l'affichage visuel du protocole de perfusion de sang, y compris l'au moins un statut d'unité de sang individuelle ;
recevoir et traiter (280) les données de balayage provenant du scanneur ; et interrompre (290) le protocole de perfusion de sang si les données de balayage traitées indiquent que l'une des unités de sang individuelles ne correspond pas à l'identifiant patient,
le protocole de perfusion de sang étant conçu pour exiger au moins deux balayages de vérification d'une unité de sang individuelle, l'unité de sang individuelle devant correspondre à l'identifiant patient pour acquérir un statut de perfusion sur l'écran pour l'unité de sang individuelle.

13. Support lisible par ordinateur selon la revendication 12, comprenant en outre les étapes consistant à :
sélectionner un niveau de service de perfusion de sang en fonction d'un paramétrage de niveau de service et/ou d'une sélection d'utilisateur,
le protocole de perfusion de sang étant déterminé par le niveau sélectionné de service de perfusion de sang et étant sélectionné parmi une pluralité de protocoles de perfusion de sang, le protocole de perfusion de sang étant conçu pour exiger un balayage de l'identifiant patient et/ou d'une unité de sang individuelle utilisée pour acquérir un statut complet sur l'écran pour l'unité de sang individuelle utilisée, et le protocole de perfusion de sang étant conçu pour fournir un accès utilisateur à la documentation de perfusion après l'acquisition d'un statut complet.

14. Support lisible par ordinateur selon la revendication 12, les étapes consistant en outre à : changer le statut de l'unité de sang individuelle en un statut vérifié sur un écran lorsque les données de balayage traitées indiquent que l'unité de sang individuelle correspond à l'identifiant patient après un premier balayage de vérification, comme indiqué par un changement de couleur et/ou un message sur l'écran.

15. Support lisible par ordinateur selon la revendication 14, la documentation de perfusion comprenant au moins l'un parmi les signes vitaux du patient, une liste de contrôle, une vérification de témoin, et le volume d'unité de sang ; les étapes consistant en outre à : nécessiter un délai temporel dans lequel la documentation de perfusion doit être complétée.
